(19)

Europäisches
Patentamt
European
Patent Office
Office européen
des brevets

(11) **EP 3 019 870 B1**

(12) **EUROPÄISCHE PATENTSCHRIFT**

(45) Veröffentlichungstag und Bekanntmachung des Hinweises auf die Patenterteilung:
**18.04.2018 Patentblatt 2018/16**

(21) Anmeldenummer: **14761560.3**

(22) Anmeldetag: **25.06.2014**

(51) Int Cl.:
***G01N 33/68*** *(2006.01)* ***B01D 15/32*** *(2006.01)*

(86) Internationale Anmeldenummer:
**PCT/DE2014/000319**

(87) Internationale Veröffentlichungsnummer:
**WO 2015/003674 (15.01.2015 Gazette 2015/02)**

(54) **VERFAHREN ZUR QUANTITATIVEN CHARAKTERISIERUNG AMYLOIDER UND/ODER AGGREGIERENDER PEPTIDE UND/ODER PROTEINE IN EINER PROBE**

METHOD FOR THE QUANTITATIVE CHARACTERISATION OF AMYLOID AND/OR AGGREGATING PEPTIDES AND/OR PROTEINS IN A SAMPLE

PROCÉDÉ DE CARACTÉRISATION QUANTITATIVE DE PEPTIDES ET/OU DE PROTÉINES AMYLOÏDES ET/OU AGRÉGÉS DANS UN ÉCHANTILLON

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **12.07.2013 DE 102013011634**

(43) Veröffentlichungstag der Anmeldung:
**18.05.2016 Patentblatt 2016/20**

(73) Patentinhaber: **Forschungszentrum Jülich GmbH 52425 Jülich (DE)**

(72) Erfinder:
• **NAGEL-STEGER, Luitgard 40764 Langenfeld (DE)**
• **BRENER, Oleksandr 40591 Düsseldorf (DE)**
• **GREMER, Lothar 41541 Dormagen-Zorn (DE)**
• **WILLBOLD, Dieter 52428 Jülich (DE)**

(56) Entgegenhaltungen:
WO-A2-2009/052837    WO-A2-2013/092951

• **DEGUO DU ET AL: "A Kinetic Aggregation Assay Allowing Selective and Sensitive Amyloid-[beta] Quantification in Cells and Tissues", BIOCHEMISTRY, Bd. 50, Nr. 10, 15. März 2011 (2011-03-15) , Seiten 1607-1617, XP055154160, ISSN: 0006-2960, DOI: 10.1021/bi1013744**
• **MICHAEL KRAMER ET AL: "Selective detection, quantification, and subcellular location of [alpha]-synuclein aggregates with a protein aggregate filtration assay", BIOTECHNIQUES, Bd. 44, Nr. 3, 1. März 2008 (2008-03-01), Seiten 403-411, XP055154158, ISSN: 0736-6205, DOI: 10.2144/000112691**
• **CHANG ET AL: "Detection and quantification of tau aggregation using a membrane filter assay", ANALYTICAL BIOCHEMISTRY, ACADEMIC PRESS INC, NEW YORK, Bd. 373, Nr. 2, 5. Januar 2008 (2008-01-05), Seiten 330-336, XP022411115, ISSN: 0003-2697, DOI: 10.1016/J.AB.2007.09.015**

**EP 3 019 870 B1**

**Beschreibung**

Die Erfindung bezieht sich auf ein Verfahren zur quantitativen Charakterisierung amyloider und/oder aggregierender Peptide und/oder Proteine in einer Probe, u. a. zur quantitativen Bestimmung des Einflusses eines Wirkstoffes auf die Konzentration, Größe und Form von Aggregaten amyloider und/oder aggregierender Peptide und/oder Proteine.

Stand der Technik

Aus Deguo et al. (Deguo Du, Amber N. Murray, Ehud Cohen, Hyun-Eui Kim, Ryan Simkovsky, Andrew Dillin, and Jeffery W. Kelly (2011). A Kinetic Aggregation Assay Allowing Selective and Sensitive Amyloid-β Quantification in Cells and Tissues. Biochemistry 50, 1607-1617) ist ein Aggregationsassay zur Abeta Quantifizierung bekannt.

Es existiert noch kein zugelassenes Medikament für eine ursächliche Behandlung der Alzheimerschen Demenz (AD). Typischerweise findet man in den Gehirnen von AD-Patienten *post-mortem* Ablagerungen des sogenannten beta-Amyloid-Peptides (Aβ) in Plaques. Deshalb werden schon lange verschiedene Formen des Aβ, z. B. Fibrillen, für die Entstehung und das Fortschreiten von AD verantwortlich gemacht.

Seit wenigen Jahren werden besonders die kleinen Aβ-Aggregate (Aβ-Oligomere) als hauptsächlicher Verursacher für die Entstehung und den Fortschritt der AD verantwortlich gemacht. Eine Reduktion oder vollständige Eliminierung von Aβ-Oligomeren wäre somit das wichtigste Kriterium, um die AD zu heilen oder zu verlangsamen.

Aβ-Monomere entstehen ständig in unserem Körper und sind vermutlich per se nicht toxisch. Es wird spekuliert, ob sich Aβ-Monomere abhängig von ihrer Konzentration, die sich letztlich durch Bildungs- und Abbau-Raten im Körper ergibt, zufällig und damit mit zunehmendem Alter immer wahrscheinlicher spontan zu Aβ-Oligomeren zusammen lagern. Einmal entstandene Aβ-Oligomere könnten sich durch einen Prion-ähnlichen Mechanismus dann vermehren und letztlich zur Krankheit führen.

Aufgrund dieser Überlegungen sollte es das Ziel einer ursächlichen Behandlung sein, toxische Aβ-Oligomere vollständig zu vernichten und/oder deren Prion-ähnliche Vermehrung zu verhindern. Ein wichtiger Punkt dabei ist, dass jeder Wirkstoff in einem Tiermodel und in klinischen Studien getestet werden muss. Diese sind sehr zeitintensiv und kostspielig. Von großem Vorteil wäre eine schnelle, zuverlässige und quantitative in vitro-Untersuchung, die die effektivsten Wirkstoffe gegen Aβ-Oligomere vorselektiert.

Vor einigen Jahren wurde ein D-enantiomeres Peptid mit dem Namen D3 durch eine Spiegelbild-Phagendisplay-Selektion gegen überwiegend monomeres Aβ(1-42) identifiziert mit dem Plan, dieses durch die Bindung zu stabilisieren und seine Umwandlung in toxische Aβ-Aggregate zu verhindern. Nach dem derzeitigen Kenntnisstand zerstört D3 die besonders toxischen Aβ-Oligomere und wandelt sie dabei in nichttoxische, nicht-amyloidogene und ThT-negative amorphe Aggregate um. Im Tiermodell wird selbst durch eine orale Verabreichung von D3 mit dem Trinkwasser erreicht, dass behandelte transgene AD-Mäuse deutlich weniger Plaques enthalten und signifikant verbesserte kognitive Fähigkeiten besitzen.

Nach dem Stand der Technik werden Aβ-Oligomere z. B. in einer Aβ-haltigen, nicht aufgetrennten Probe mittels Oligomer-spezifischer Antikörper detektiert und quantifiziert. Diese Methode ist nur semi-quantitativ, weil man für jede zu bestimmende Probe einen Vergleichsstandard benötigt, der notwendigerweise gleichzeitig mit der zu messenden Probe im Assay mitgeführt werden muss. Außerdem ist diese Methode nicht zuverlässig, weil die Oligomer-spezifischen Antikörper möglicherweise nicht alle Oligomer-Arten erkennen oder diese nicht in gleichem Maße erkennen.

Des Weiteren kann man mit Hilfe unterschiedlicher Zentrifugationstechniken eine Aβ-haltige Probe fraktionieren, so dass in unterschiedlichen Fraktionen unterschiedliche Aβ-Spezies vorliegen. Diese kann man anschließend mittels ELISA, Western Blot oder SDS-PAGE analysieren, wie z. B. aus Funke et al. bekannt ist (S. A. Funke, T. van Groen, I. Kadish, D. Bartnik, L. Nagel-Steger, O. Brener, T. Sehl, R. Batra-Safferling, C. Moriscot, G. Schoehn, A. H. C. Horn, A. Muller-Schiffmann, C. Korth, H. Sticht, D. Willbold. Oral Treatment with the D-Enantiomeric Peptide D3 Improves the Pathology and Behavior of Alzheimer's Disease Transgenic Mice. ACS Chem. Neurosci. (2010), 1, 639-648).

Eine dritte Methode, die oft verwendet wird, ist der ThioflavinT (ThT)-Test, mit dem aber nachteilig nur eine Reduzierung des Fibrillen-Anteils gemessen werden kann. Dies reicht nach heutigem Wissen nicht aus, um einen erfolgversprechenden Wirkstoffkandidaten für die Oligomer-Reduzierung zu identifizieren.

Somit sind nachteilig alle auf Antikörperdetektion basierenden Techniken von der Zugänglichkeit des Epitopes abhängig. Aufgrund verschiedener Aβ-Aggregatstrukturen sind die Epitope aber öfters verdeckt. SDS-Polyacrylamid-Gelelektrophorese (SDS-PAGE-) Analysen sind zwar von den oben genannten Problemen unabhängig, haben aber nachteilig eine geringere Sensitivität und sind nicht quantitativ. SDS-PAGE ist zudem nachteilig, da verschiedene Banden aus Dimeren, Trimeren und Tetrameren gebildet und nachgewiesen werden, da insbesondere die stark aggregierenden Proben hochmolekulare Aggregate bilden. Somit ist auch dieses Verfahren nachteilig nur semi-quantitativ, weil man für jede zu bestimmende Probe einen Vergleichsstandard benötigt, der notwendigerweise gleichzeitig mit der zu messenden Probe im Assay mitgeführt werden muss.

Aufgabe der Erfindung

Aufgabe der Erfindung ist es ein Verfahren zur quantitativen Charakterisierung amyloider und/oder aggregierender Peptide und/oder Proteine in einer Probe bereit zu stellen.

Lösung der Aufgabe

Die Aufgabe wird gelöst nach dem Verfahren nach Patentanspruch 1. Vorteilhafte Ausgestaltungen hierzu ergeben sich aus den hierauf rückbezogenen Patentansprüchen.

Beschreibung der Erfindung

Das Verfahren zur quantitativen Charakterisierung amyloider und/oder aggregierender Peptide und/oder Proteine in einer Probe, weist nachfolgende Schritte auf:

-   eine Probe wird bereit gestellt, wobei die Probe ein amyloides und/oder aggregierendes Peptid und/oder Protein in mindestens einer Aggregatgröße und -Form umfasst,

-   ein zu untersuchender Wirkstoff wird zu der Probenlösung zugegeben,

-   die amyloiden und/oder aggregierenden Peptide und/oder Proteine werden in Hinblick auf ihre Aggregatgröße und -Form durch Dichtegradientenzentrifugation als Fraktionierung voneinander getrennt. Hierdurch wird aus der Probe eine Mehrzahl an in Hinblick auf die Konzentration untersuchbaren Fraktionen erhalten, in denen die amyloiden und/oder aggregierenden Peptide und/oder Proteine mit einer bestimmten Aggregatgröße und -Form vorliegen,

-   die amyloiden und/oder aggregierenden Peptide und/oder Proteine einer bestimmten Fraktion werden optional vollständig zu Monomerbausteinen denaturiert,

-   die Konzentrationsänderung der Peptid- und/oder Protein- Bausteine wird durch einen Vergleich mit Kontrollwerten ohne den Wirkstoff bestimmt.

Die Aufgabe wird ebenfalls durch nachfolgendes Verfahren gelöst.
Das Verfahren zur quantitativen Charakterisierung amyloider und/oder aggregierender Peptide und/oder Proteine in einer Probe, weist hierzu nachfolgende Schritte auf:

-   eine Probe wird bereit gestellt, wobei die Probe ein amyloides und/oder aggregierendes Peptid und/oder Protein in mindestens einer Aggregatgröße und -Form umfasst,

-   ein zu untersuchender Wirkstoff wird zu der Probenlösung zugegeben,

-   die amyloiden und/oder aggregierenden Peptide und/oder Proteine werden im Hinblick auf ihre Aggregatgröße und -Form durch Dichtegradientenzentrifugation als Fraktionierung voneinander zu mehreren, mindestens 2, vorzugsweise mindestens 3, besonders bevorzugt mindestens 4, 5, 6, 7, 8, 9, 10, 11, 12, 13, 14 oder 15 untersuchbaren Fraktionen getrennt. Hierdurch wird aus der Probe

-   eine Mehrzahl an in Hinblick auf die Konzentration untersuchbaren Fraktionen erhalten, in denen die amyloiden und/oder aggregierenden Peptide und/oder Proteine mit einer bestimmten Aggregatgröße und -Form vorliegen,

-   die amyloiden und/oder aggregierenden Peptide und/oder Proteine einer bestimmten Fraktion werden optional vollständig zu Monomerbausteinen denaturiert,

-   die Konzentrationsänderung der Peptid- und/oder Proteinbausteine in mindestens einer Fraktion, vorzugsweise in mindestens 2, 3, 4, 5, 6, 7, 8, 9, 10, 11, 12, 13, 14 oder aber 15 in Hinblick auf die Konzentration untersuchbaren Fraktionen wird durch einen Vergleich mit Kontrollwerten ohne den Wirkstoff bestimmt.

Es kann vorteilhaft durch eines der oben genannten Verfahren jede gewünschte Fraktion auf Konzentrationsänderungen nach Wirkstoffzugabe untersucht werden. Das Verfahren stellt somit einen Verfahrensschritt bereit, der es erlaubt mehr als eine Fraktion auf Konzentrationsänderungen hin zu untersuchen oder auch auf Änderungen der Aggregatgröße oder anderer Parameter. Somit werden durch das Verfahren Fraktionen gewonnen, die sowohl in quantitativer als in qualitativer Weise untersucht werden können und nicht nur in Hinblick auf Konzentrationsänderungen. Der Begriff "gewünschte Fraktion" im Sinne des Verfahrens umfasst insbesondere aber nicht ausschließlich solche Fraktionen, die vor der Auftrennung auch aggregierende bzw. aggregierte Peptide und/oder Proteinbausteine enthielten, insbesondere toxische Oligomere.

Zunächst wird eine Probenlösung vorbereitet, die amyloide und/oder aggregierende Peptide und/oder Proteine umfasst. Es kann auch eine Probe aus einem anderen Experiment, einer Zellkultur, einem Tier oder einem Menschen entnommen werden. Die Peptide und/oder Proteine weisen ein Aggregationsverhalten auf. Dies heißt, dass unter bestimmten Voraussetzungen die Peptide und/oder Proteine miteinander zu höher molekularen Formen aggregieren. Beispielhaft sei das bei der Alzheimerschen Demenz auftretende A$\beta$(1-42)-Peptid genannt, welches eine besonders starke Tendenz zur Aggregation aufweist. Neben A$\beta$(1-42) treten auch noch andere Varianten von A$\beta$ auf, z. B. A$\beta$(1-40), Aß(1-43), A$\beta$(3-40), A$\beta$(1-40), pyroGlu-A$\beta$(3-40) und pyroGluA$\beta$(3-42) auf.

Es wird z. B. ein cytotoxisches, oligomeres Assembly des (A$\beta$-)Peptids und/oder Proteins angereichert oder bereit gestellt, wobei eine spezifische Größenverteilung durch eine Vorinkubation eingestellt wird. Dies bewirkt vorteilhaft, dass der zu untersuchende Wirkstoff hinsichtlich seines Einflusses auf die Partikelgrößenverteilung und gegebenenfalls Formverteilung exakt und eindeutig quantitativ untersucht werden kann. Selbst im Falle der aggregierenden A$\beta$(1-42) umfassenden Proben kann auf diese Weise schnell der Einfluss des Wirkstoffes auf die toxischen Aß-Oligomer-Spezies in der Probe bestimmt werden.

Es wird ein Wirkstoff zu der Probe zugegeben, welche die amyloiden und/oder aggregierenden Peptide und/oder Proteine mit unterschiedlicher Aggregatgröße und -Form umfasst. Der Wirkstoff ändert die Größenverteilung und damit die Konzentration bestimmter Aggregate in der Probe. Diese Konzentrationsänderung wird quantitativ festgestellt. Die Änderung ist ein Maß für die Reduktion oder sogar für die vollständige Eliminierung bestimmter toxischer Spezies mit nachweisbarer Aggregat- bzw. Partikelgröße. Das heißt, dass während des Verfahrens die Zunahme oder die Abnahme der Konzentration bestimmter amyloider und/oder aggregierender Peptide und/oder Proteine über die Änderung der Aggregatgrößenverteilung nachgewiesen wird.

Für ein vorzunehmendes Screening werden mehrere Wirkstoffe und deren Einfluss auf die Probe untersucht. Dann wird vorteilhaft ein Verfahren für eine schnelle Vorselektionierung geeigneter Wirkstoffe bereitgestellt.

Die Zusammensetzung an amyloiden und/oder aggregierenden Peptiden und/oder Proteinen mit unterschiedlicher Aggregatgröße und -Form wird also während des Verfahrens unter Einfluss des Wirkstoffs verändert. Während einige amyloide und/oder aggregierende Peptide und/oder Proteine mit einer bestimmten Größe anfänglich in der Probe vorhanden waren, werden diese unter Einfluss des Wirkstoffs reduziert oder sogar vorteilhaft vollständig eliminiert. Andere Partikelgrößen nehmen unter dem Einfluss des Wirkstoffs zu oder bleiben konstant.

Die Partikel, die aus den amyloiden und/oder aggregierenden Peptiden und/oder Proteinen gebildet sind, werden voneinander getrennt.

Auf diese Weise wird vorteilhaft bewirkt, dass aus der Probe eine Mehrzahl an Fraktionen erhalten wird. In den Fraktionen sind die Partikel an amyloiden und/oder aggregierenden Peptiden und/oder Proteinen mit jeweils einer bestimmten Aggregatgröße und -Form enthalten. Diese Trennung der Partikel wird mittels einer Dichtegradientenzentrifugation nach dem s-Wert vorgenommen. Andere physikalische Parameter der Aggregate können ebenfalls als Grundlage der vorzunehmenden Fraktionierung herangezogen werden, z. B. der hydrodynamische Radius der Partikel. Die Fraktionen werden räumlich voneinander getrennt, z. B. durch Abpipettieren. Die Dichtegradientenzentrifugation hat den Vorteil alle ursprünglich in der Probe vorhandenen Aggregate der amyloiden und/oder aggregierenden Peptide und/oder Proteine für eine weitere quantitative Analyse bereit zu stellen.

Abschließend wird die Konzentration an amyloiden und/oder aggregierenden Peptiden und/oder Proteinen in der jeweiligen Fraktion bestimmt.

Dadurch wird vorteilhaft die Konzentrationsänderung an amyloiden und/oder aggregierenden Peptiden und/oder Proteinen mit jeweils bestimmter Größe quantitativ unter dem Einfluss des Wirkstoffs ermittelt. Durch einen Vergleich mit Kontrollen ohne den Wirkstoff kann der Einfluss des Wirkstoffes auf die Verteilung der Aggregate der amyloiden und/oder aggregierenden Peptide und/oder Proteine in der jeweiligen Fraktion quantitativ bestimmt werden. Hierdurch erhält man ein Maß unter anderem über die Wirksamkeit des Wirkstoffes hinsichtlich seiner Fähigkeit bestimmte Spezies aus der Probe zu eliminieren, z. B. toxische Oligomere.

Es ist möglich, eine einzige Fraktion auf diese Weise zu untersuchen. Man erhält dann mit dem erfindungsgemäßen Verfahren ein Maß über die Fähigkeit des Wirkstoffs die bestimmten Konformere aus der Fraktion quantitativ zu verändern, z. B. toxische Oligomere aus der Probe zu eliminieren.

Vorteilhaft wird so ein Verfahren bereitgestellt, bei dem die Änderung des Anteils an Monomeren und/oder Oligomeren und / oder Fibrillen und / oder besonders großen Aggregaten unter dem Einfluss des Wirkstoffes quantitativ bestimmt werden kann.

In einer Ausgestaltung der Erfindung wird auch die Änderung der Formverteilung der amyloiden und/oder aggregierenden Peptide und/oder Proteine unter dem Einfluss des Wirkstoffes untersucht, z. B. durch Kraftfeldmikroskopie. Auf diese Weise wird vorteilhaft neben der Änderung der Partikelgrößenverteilung auch die Änderung der Formverteilung der amyloiden und/oder aggregierenden Peptide und/oder Proteine unter dem Einfluss des Wirkstoffs erfasst, überprüft und/oder bestätigt.

Vorzugsweise werden mehrere Wirkstoffe in einem Screeningverfahren hinsichtlich des Einflusses auf die Partikelgrößenverteilung der Probe *in vitro* getestet. Ganz besonders vorteilhaft ist das Verfahren geeignet für das Screening potentieller Wirkstoffe gegen die Alzheimersche Demenz (AD) basierend auf der Modulation der toxischen Amyloid-$\beta$ (A$\beta$)-Oligomere unter dem Einfluss des Wirkstoffes.

Das erfindungsgemäße Verfahren liefert zudem ein umfassendes, quantitatives Ergebnis in Hinblick auf die sich ändernde Partikel- bzw. Aggregatgrößenverteilung amyloider und/oder aggregierender Peptide und/oder Proteine unter dem Einfluss des Wirkstoffes. Damit werden die erfolgversprechendsten Wirkstoffe, z. B. für eine Therapie der Alzheimerschen Demenz vorselektioniert, welche die Konzentration an löslichen toxischen Bestandteilen wie z. B. den A$\beta$-Oligomeren verringern.

Das Verfahren ist hierauf nicht beschränkt. Ohne eine Einschränkung des Verfahrens erlaubt dieses darüber hinaus auch die Feststellung, ob der Wirkstoff zu einer Zunahme anderer potentiell toxischer oder erwünschter Spezies in der Probe führt. Hierzu zählen im Falle der Alzheimerschen Demenz z. B. die A$\beta$(1-42)-Monomere und/oder -Fibrillen. Vorzugsweise wird das Verfahren angewendet bei der Ermittlung von Wirkstoffen die nach heutigem Erkenntnisstand nicht zu einer Zunahme anderer toxischer Bestandteile führen. Hierzu werden besonders vorteilhaft mehrere der erhaltenen Fraktionen auf Konzentrationsänderung der Bausteine erfindungsgemäß untersucht.

In einer besonders vorteilhaften Ausgestaltung der Erfindung wird eine Probe vorbereitet, welche wenig oder gar keine Fibrillen und/oder größere Aggregate umfasst. Auf diese Weise wird vorteilhaft bewirkt, dass deren Bildung unter Einfluss des Wirkstoffes besonders einfach nachgewiesen werden kann.

Die Fraktionierung der in der Probenlösung befindlichen amyloiden und/oder aggregierenden Peptide und/oder Proteine erfolgt mittels einer Dichtegradientenzentrifugation unter Verwendung von z. B. Optiprep, Percoll, Saccharose oder analogem Dichtegadientenmaterial. Die Aggregate werden dabei nach der Größe und gegebenenfalls der Form (Sedimentationskoeffizient) voneinander getrennt. Dieses Verfahren ist besonders bei aggregierenden A$\beta$(1-42) Aggregaten und Tau-Aggregaten vorteilhaft.

Der Begriff "exakt bestimmt" umfasst besonders vorteilhaft einen Kalibrierungsschritt bei der Fraktionierung durch Moleküle bekannter Art und Verhaltens. Nach der Fraktionierung liegt in jeder Fraktion nur eine bestimmte (das heißt bekannte) Art an Konformer vor, z. B. Oligomere oder Fibrillen und so weiter.

Bei der Dichtegradientenzentrifugation als Fraktionierungsschritt werden die Konformere nach ihrem s-Wert oder Sedimentationskoeffizienten aufgetrennt. Moleküle unterschiedlicher Größe können einen identischen hydrodynamischen Radius besitzen, haben aber dennoch unterschiedliche s-Werte und werden danach auch aufgetrennt. Durch eine Kalibration mit Molekülen von bekanntem s-Wert sind die mittels Dichtegradientenzentrifugation erhaltenen Aggregate exakt nach ihrem s-Wert bestimmt.

Die Konzentrationsänderung an amyloiden und/oder aggregierenden Peptiden und/oder Proteinen mit einer bestimmten Größe in einer jeweiligen Fraktion wird in einer ganz besonders vorteilhaften Ausgestaltung der Erfindung nach der Zugabe des Wirkstoffs durch eine vollständige Denaturierung der amyloiden Peptide und/oder Proteinbausteine während einer im Anschluss an die Fraktionierung durchgeführten Umkehrphasen-(RP-) HPLC bestimmt.

Eine vollständige Denaturierung kann allgemein gesprochen über eine physikochemische Reaktion mit einem denaturierenden Agenz oder über die Temperatur erfolgen. Auf diese Weise wird vorteilhaft bewirkt, dass ausschließlich die Monomer Bausteine der quantitativen Analyse zugeführt werden.

In einer ganz besonders vorteilhaften Ausgestaltung der Erfindung erfolgt der Schritt der Denaturierung quasi per se durch die Säulenchemie und Temperatur. Hierdurch werden Schritte bei der Aufarbeitung der Probe eingespart, so dass das erfindungsgemäße Verfahren sehr schnell durchgeführt werden kann.

Grundsätzlich lässt sich die Konzentration der amyloiden und/oder aggregierenden Peptide und/oder Proteine in der jeweiligen Fraktion aber auch durch Isotopenmarkierung des verwendeten Peptids und/oder Proteins und anschließend an die Fraktionierung erfolgender Szintillationszählung der Fraktionen bestimmen.

Um festzustellen, welchen Einfluss die Wirkstoffzugabe auf vorhandene Oligomere hatte, wird die Lösung daher zunächst inkubiert und vor oder nach der Wirkstoffzugabe dann vorzugsweise auf einen vorgeformten Dichtegradienten aufgeschichtet und die darin enthaltenen Aggregat-Partikel entsprechend durch Ultrazentrifugation getrennt. Dabei spielt die Partikelgröße eine wichtige Rolle. Im Laufe dieser Zentrifugation sind auf diese Weise z. B. unterschiedliche A$\beta$-Aggregate, wie Monomere, Oligomere und Fibrillen oder amorphe Aggregate nach ihrem unter anderem von der Partikelgröße abhängigen Sedimentationskoeffizienten voneinander getrennt. Diese werden fraktioniert geerntet.

Bei der Dichtegradientenzentrifugation werden die unterschiedlichen Aggregate nach ihrem s-Wert voneinander getrennt. Je größer der Partikel ist, desto weiter wandert er in den Gradienten ein. In den geernteten Fraktionen wird die Konzentration des (A$\beta$-) amyloiden und/oder aggregierenden Peptids und/oder Proteins mittels RP-HPLC bestimmt.

Dazu wird die Fraktion des Gradienten, welche unter den verwendeten Zentrifugationsbedingungen Partikel mit entsprechender Größe enthält, vorzugsweise komplett denaturiert und durch RP-HPLC analysiert.

Die Denaturierung der Aggregate zu Monomeren kann z. B. auf einem RP-HPLC-Säulenmaterial wie z. B. einer C8-Säule mit etwa 30 % (v/v) Acetonitril und 0,1 % (v/v) Trifluoressigsäure als mobiler HPLC-Phase bei einer Säulentemperatur von etwa 80 °C vollständig erfolgen. Die entstehenden Monomere des amyloiden und/oder aggregierenden Peptids und/oder Proteins werden dann vorzugsweise durch RP-HPLC nach Hydrophobizität aufgetrennt. Dadurch werden vorteilhaft auch Aß-Spezies vom Dichtegradientenmaterial Iodixanol, welches aufgrund seiner spektroskopischen Eigenschaften die Absorptionsmessung von Proteinen unmöglich macht, getrennt. Eluierendes Peptid, wie z. B. A$\beta$, wird mittels der UV-Absorption bei 215 nm detektiert. Die Peakflächenintegration kann mittels Agilent Chemstation Software erfolgen. Die Verrechnung daraus entstandener Werte mit der zuvor durchgeführten Kalibrierung erlaubt die Berechnung der Konzentration des in der jeweiligen Fraktion vorhandenen Peptids bzw. Proteins. Je Fraktion sollte der Mittelwert aus mehreren z. B. sechs voneinander unabhängig durchgeführten Experimenten mit der sich ergebenen Standardabweichung berechnet werden.

Der Vorteil der HPLC-Analyse liegt darin, dass man dann z. B. A$\beta$ unabhängig vom vorherigen Aggregationszustand sehr sensitiv detektieren (z. B. ca. 20 nM oder 1,8 ng A$\beta$(1-42)) und zuverlässig quantifizieren kann.

Ein entscheidender Vorteil des erfindungsgemäßen Verfahrens kann daher in einer Kopplung von Dichtegradientenzentrifugation und RP-HPLC bestehen, welche eine zuverlässige Quantifizierung insbesondere auch von A$\beta$-Oligomeren ermöglicht. Hierzu wird in einer vorteilhaften Ausgestaltung der Erfindung die Fraktion, umfassend toxische Oligomere mit und ohne Wirkstoffzugabe und ihre Veränderung der Konzentration untersucht.

Überraschend wurde im Rahmen der Erfindung erkannt, dass die während der RP-HPLC verwendete mobile Phase in Kombination mit der erhöhten Säulentemperatur eine vollständige Denaturierung der in allen möglichen Aggregationszuständen vorliegenden A$\beta$-Spezies gewährleistet. Ein Fachmann kann die Chemie und Temperatur der Bedingungen dieses Verfahrensschrittes anpassen, so dass die gewünschte Denaturierung der Probe erfolgt.

Diese Erkenntnis ist insofern überraschend, da einige der bei der Alzheimerschen Demenz auftretenden A$\beta$-Spezies besonders hohe Tendenzen zur Aggregation zeigen. Auch in diesem Fall ist aber die Denaturierung der amyloiden und/oder aggregierenden Peptide und/oder Proteine zu Monomer gewährleistet.

Besonders überraschend ist zudem, dass mit einer RP-HPLC auch das durch Denaturierung als Monomer vorliegende amyloide und/oder aggregierende Peptid und/oder Protein von der den Dichtegradienten ausbildenden Substanz sauber getrennt wird und der quantitativen Analyse reproduzierbar zugänglich gemacht wird.

Durch die besonders vorteilhafte Kombination einer Dichtegradientenzentrifugatio basierenden Fraktionierung und der Konzentrationsbestimmung mit RP-HPLC wurde somit ein Verfahren entwickelt, welches besonders schnell den Einfluss potentieller Wirkstoffe auf den Anteil an toxischen Oligomer-Spezies eines A$\beta$(1-42)-Peptids oder anderer Peptide und/oder Proteine quantifiziert, da es dabei auf die Monomerform abstellt.

Ein Vergleich der Kontrolle mit der einen Wirkstoff oder einen natürlichen Liganden beinhaltenden Probe erlaubt eine reproduzierbare und schnelle Bestimmung der Wirkstoffeffektivität bezüglich der Eliminierung und Reduktion bestimmter Spezies wie z. B. Oligomere und somit eine Abschätzung seiner Wirkung in einem Tiermodel und später in den klinischen Testphasen. Im Gegensatz hierzu ist gemäß des Stands der Technik nur die Möglichkeit beschrieben, den Einfluss einer Substanz auf den A$\beta$-Oligomer Gehalt zu quantifizieren.

Ganz besonders vorteilhaft erhält man gleichzeitig aber auch durch entsprechende HPLC-Analyse und -Quantifizierung auch Daten zum Einfluss, den der Wirkstoff auf die Zunahme des amyloiden und/oder aggregierenden Peptids und/oder Proteins in anderen Fraktionen hat, z. B. auf A$\beta$-Monomere und/oder Fibrillen enthaltende Fraktionen.

Es ist denkbar, dass mit dem erfindungsgemäßen Verfahren eine Vielzahl an potentiellen Wirkstoffen hinsichtlich ihres Einflusses auf die Partikelgrößen-Verteilung amyloider und/oder aggregierender Peptide und/oder Proteine in einer Probe schnell und reproduzierbar quantifiziert werden. Die Probe kann dabei synthetischer Art sein. Es können aber auch natürliche Wirkstoffe oder entnommene Proben auf diese Weise untersucht werden.

Ausführungsbeispiele

Im Weiteren wird die Erfindung an Hand von Ausführungsbeispielen und der beigefügten Figuren näher erläutert, ohne dass es hierdurch zu einer Beschränkung der Erfindung kommen soll.

Das erfindungsgemäße Verfahren wurde angewendet, um den Effekt von D3 (D-Enantiomer) gemäß SEQ ID NO: 1 auf Aß-Oligomere zu quantifizieren. Zu Überprüfung in einem Screening wurde ein Dimer von D3 synthetisiert und im oben beschriebenen Assay eingesetzt, um zu überprüfen, ob D3D3 (D-Enantiomer) tatsächlich noch effizienter als D3 ist.

Für die nachfolgenden Ausführungsbeispiele wurden lediglich eine Ultrazentrifuge und eine HPLC-Anlage mit passender Trennsäule und einem UV-Detektor benötigt. Zur Ermittlung der Reduzierung des A$\beta$-Oligomer-Anteils in einer Aggregatmischung wurde vorbehandeltes und getrocknetes A$\beta$(1-42)-Peptid in Puffer gelöst und so lange inkubiert, dass ein möglichst großer A$\beta$-Oligomer-Anteil entstand. Zu dieser mit Oligomeren angereicherten Probe wurde entsprechend der zu analysierende Wirkstoff zugegeben.

Es zeigen:

Figur 1: Säulenchromatogramme einer RP-HPLC für unterschiedliche Aggregationszustände des Aβ(1-42)-Peptids.

Figur 2: Chromatogramme der RP-HPLC für die Fraktionen 1 bis 15 eines Dichtegradienten mit den daraus berechneten Konzentrationen je Fraktion.

Figur 3: Gemittelte Größenverteilung für sechs Aβ(1-42)-Kontrollen ohne Wirkstoffzusatz nach Dichtegradientenzentrifugation und RP-HPLC.

Figur 4: Anwendung des vorgestellten Verfahrens auf die Analyse potentieller Wirkstoffe für die Therapie der Alzheimerschen Demenz am Beispiel eines D-Peptids (D3) und seines Tandem-Dimers (D3D3).

Figur 5: Versuchsansatz zur quantitativen Charakterisierung von amyloidem und/oder aggregierendem Aβ ohne und mit Wirkstoff, sowie Festellung der Änderung der Konzentration von Monomeren in verschie-denen Fraktionen nach Denaturierung während und mit der RP-HPLC und Wirkstoffzugabe.

Material und Methoden

1 mg lyophilisiertes Aβ(1-42) (Bachem, Heidelberg) wurde nach der Entnahme aus dem Gefrierschrank für 10 bis 30 min bei Raumtemperatur (RT) inkubiert, um die Temperatur des Peptidpellets und der Umgebung an einander anzugleichen und somit die Einkondensation von Luftfeuchtigkeit zu vermeiden. Danach wurde das Aß-Peptid in HFIP (1,1,1,3,3,3-Hexafluoro-2-Propanol) (Sigma-Aldrich, Taufkirchen) in einer Konzentration von 1 mg/700 μl gelöst und zur vollständigen Lösung über Nacht unter Schütteln bei RT inkubiert, um sicher zu stellen, dass zu diesem Zeitpunkt das Aβ(1-42) vollständig in Monomere aufgespalten vorliegt. Anschließend wurde das Aβ(1-42) je 36 μg pro Eppendorfgefäß aliquotiert. Um das HFIP zu entfernen, wurden die Aliquots unter dem Abzug für ca. 30 min offen stehen gelassen. Der Rotations-Vakuumkonzentrator (Rotations-Vakuumkonzentrator RVC 2-18, Christ, Mainz-Mombach) wurde ca. 30 min vorgekühlt. Danach wurden die Aβ(1-42)-Aliquots 30 min lang im Rotations-Vakuumkonzentrator nachgetrocknet, damit auch die letzten HFIP-Reste abdampfen konnten. Das aliquotierte, getrocknete Aβ(1-42) wurde bis zur Verwendung bei -20 °C gelagert.

Vor dem Experiment wurde ein Aβ(1-42)-Aliquot in 100 μl 10 mM $NaH_2PO_4$/$Na_2HPO_4$-Puffer (Sigma-Aldrich, Taufkirchen), pH 7,4 zu 80 μM gelöst und unter Schütteln bei RT für 6 h inkubiert. Die Inkubationszeit von 6 h wurde für dieses spezielle Aβ dieser speziellen Firma optimiert, um sicher zu stellen, dass ein möglichst großer Anteil des Aβ als Oligomer vorliegt, um dessen Verringerung möglichst eindeutig messen zu können. Für andere Aβ-Peptide anderer Herstellerfirmen kann die Inkubationszeit variieren und soll/kann entsprechend angepasst werden.

Danach wurde der zu analysierende Wirkstoffkandidat zu 10 oder 20 μM zugegeben. Die Koinkubation der Aß-Wirkstoff-Lösung war hier stets unter Schütteln bei RT 40 min. Falls kein Wirkstoff zugegeben wurde (Kontrolle), wurde die Aß-Lösung auf die gleiche Weise behandelt.

Anschließend wurden die Proben auf einen vorgeformten Dichtegradienten (siehe Tabelle 1) aufgeschichtet. Das verwendete Gradientenmaterial bestand aus einer 60 %igen (w/v) Iodixanollösung mit dem Namen Optiprep™ (Axis-Shield, Oslo, Norwegen). Es ist eine dimere Verbindung des Iohexols mit dem systematischen Namen 5,5-[(2-hydroxy-1,3-propandiyl)-bis(acetylimino)]bis-[N,N'-bis(2,3hydroxypropyl)-2,4,6-triiodo-benzendicarboxamid]. Die Herstellung des diskontinuierlichen Gradienten erfolgte, indem auf den Boden eines Polyallomer-Zentrifugen-Röhrchens (Beckman-Coulter, Palo Alto, USA) mit den Abmessungen 11 x 34 mm die dichteste Optiprep-Verdünnung pipettiert und stufenweise mit den weniger dichten Verdünnungen überschichtet wurde. Der auf 10 mM $NaH_2PO_4$/$Na_2HPO_4$-Puffer, pH 7,4 eingestellte Gradient wurde gemäß der Tabelle 1 aus sechs Stufen aufgebaut.

Tab. 1: Volumina, Konzentration und Dichte der Gradientenstufen.

| Volumen (μl) | Iodixanol (%, w/v) | Dichte (g/ml) |
| --- | --- | --- |
| 100 | 5 | 1,030 |
| 260 | 10 | 1,056 |
| 780 | 20 | 1,109 |
| 260 | 30 | 1,162 |
| 260 | 40 | 1,215 |
| 260 | 50 | 1,267 |

Der nicht lineare Dichtegradient wurde bei 4 °C und 259.000 x g in einem TLS-55-Ausschwingrotor (Beckman-Coulter, Palo Alto, USA) für 3 h in einer TL 100-Ultrazentrifuge (Beckman-Coulter, Palo Alto, USA) zentrifugiert. Durch refraktometrische Bestimmung der Iodixanolkonzentration in den einzelnen Fraktionen wird der Dichtegradient vermessen. Eine Abschätzung der Größe von sedimentierten Proteinpartikeln anhand der Position im Gradienten am Ende der Zentrifugation erfolgt anhand einer Kalibrierung mit Markerproteinen bekannter Größe und Form, welche unter identischen Zentrifugationsbedingungen auf dem Gradienten analysiert wurden.

Das Ernten der Fraktionen erfolgte von oben nach unten durch vorsichtiges Abpipettieren. Man erhielt 14 Fraktionen von je 140 $\mu$l. Somit war die 1. Fraktion die mit der geringsten und die 14. Fraktion die mit der höchsten Dichte. Möglicherweise pelletiertes Protein wurde in die Analyse miteinbezogen. In das abgeerntete Zentrifugenröhrchen wurden 60 $\mu$l 6 M Guanidiniumchlorid (GdnCl) (Sigma-Aldrich, Taufkirchen) gegeben und 10 min bei 98 °C gekocht. Das auf diese Weise aufbereitete Pellet stellte die 15. Fraktion dar.

Nach dem Fraktionieren des Zentrifugenröhrchen-Inhaltes wurde jede Fraktion mittels RP-HPLC (HPLC-System Agilent 1260 Infinity; quarternäre HPLC Pumpe mit Solvens-Entgaser G1311B, Säulentemperiereinheit G1316A, Multiwellenlängendetektor G1365C, manueller Injektor G1328C; Agilent, Waldbronn) analysiert. Als stationäre Phase wurde eine Zorbax SB-300 C8-Säule (5 $\mu$, 4,8 x 250 mm, Agilent, Waldbronn) verwendet. Die Trennung der Substanzen erfolgte isokratisch mit 30 % (v/v) Acetonitril, 0,1 % (v/v) Trifluoressigsäure in $H_2O$ als mobiler Phase mit einer Flussrate von 1 ml/min bei einer Säulentemperatur von 80 °C. Das Probenvolumen betrug 20 $\mu$l. Eluierende Substanzen wurden mittels UV-Absorption bei 215 nm detektiert. Datenaufzeichnung und Peakflächenintegration erfolgte mittels Agilent Chemstation Software (Agilent, Waldbronn). Durch die Verwendung der RP-HPLC wird das in den Fraktionen vorhandene Dichtegradientenmaterial Iodixanol von dem A$\beta$(1-42)-Peptid getrennt. Erst durch diese Trennung ist es möglich A$\beta$ spektroskopisch zu detektieren, da ohne Abtrennung die starke Absorption des Iodixanols im UV-Bereich die Absorption des A$\beta$-Peptids überdeckt.

Die Bestimmung der molaren A$\beta$-Konzentrationen erfolgte mittels einer Kalibrierung der Säule mit A$\beta$-Lösungen mit bekannten Konzentrationen (A$\beta$-Verdünnungsreihe). Die für den linearen Zusammenhang zwischen Fläche und Peptid-Konzentration angepasste Regressionsgerade hat ein Bestimmtheitsmass $R^2$ von 0.998. Aufgrund dieser Quantifizierung ist es möglich für jeden Lauf die Rückgewinnungsrate des aufgetragenen A$\beta$42 Peptids zu berechnen. Zu diesem Zweck werden die pro Fraktion bestimmten molaren Konzentrationen durch Multiplikation mit dem Fraktionsvolumen $140 \times 10^{-6}$ l (Fraktionen 1 bis 14) und $120 \times 10^{-6}$ l (Fraktion 15) in mol/Fraktion umgerechnet aufsummiert. Der Quotient aus dieser Summe und der Anzahl der in der aufgetragenen Probe enthaltenen Mole A$\beta$-Peptid ergibt in Prozent dargestellt die Rückgewinnungsrate R.

$$R = \frac{c_P V_P + \sum_{n=1}^{14} c_n \cdot V_F}{c_0 \cdot V_0}$$

Mit $c_p$ Molare Konzentration A$\beta$ im Pellet, $c_n$ Molare Konzentration A$\beta$ in den Fraktionen 1 bis14, $c_0$ Molare Konzentration A$\beta$ vor DGZ, $V_F$ Fraktionsvolumen ($140 \times 10^{-6}$l), $V_P$ Volumen der Pelletfraktion ($120 \times 10^{-6}$l) und $V_0$ Probenvolumen vor DGZ ($100 \times 10^{-6}$l).

Die Rückgewinnungsrate betrug zwischen 0,8 und 1,0, bzw. zwischen 80 und 100 %. Durch die hohe Rückgewinnungsrate ist garantiert, dass die gewonnenen Daten den Zustand der gesamten Probe wieder spiegeln und nicht nur einen Anteil der Probe beschreiben.

Der Vorteil der RP-HPLC Methode gegenüber anderen Methoden besteht darin, dass die Quantifizierung des Abeta-Peptids unabhängig von seinem vorherigen Aggregationszustand, d. h. Monomer, Oligomer oder Fibrille, ist. Dies ist z. B. nicht bei immunochemischen Nachweismethoden garantiert, da durch die Aggregation die Antikörper-bindenden Epitope möglicherweise schlechter oder gar nicht mehr zugängig sind.

Es wurde gezeigt, dass die Denaturierung der irgendwie in der Probe vorliegenden A$\beta$ 1-42 Partikel immerzu Monomeren führt. Diese sind unabhängig von der ursprünglichen Struktur besonders vorteilhaft quantifizierbar. Hierdurch werden zuverlässig die verschiedenen Spezies wie Oligomere, Fibrillen und amorphe Aggregate immer als Monomer reproduzierbar quantifiziert.

Ergebnisse

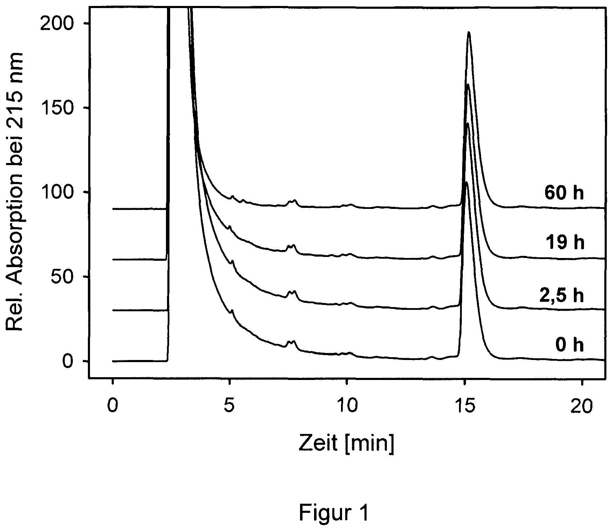

Figur 1 zeigt Säulenchromatogramme einer RP-HPLC für unterschiedliche Aggregationszustände des A$\beta$(1-42)-Peptids. Je Probe wurden 20 $\mu$l Lösung mit 1.8 ng A$\beta$(1-42) auf eine C8-Säule (5 $\mu$, 4,8 x 250 mm) injiziert und mit 30 % (v/v) Acetonitril, 0,1 % (v/v) Trifluoressigsäure in $H_2O$ als Eluent mit einer Flussrate von 1 ml/min bei einer Säulentemperatur von 80 °C eluiert. Aufgetragen ist die relative Absorption bei 215 nm des Eluats gegenüber der Retentionszeit für frisch gelöstes (0 h), 2,5 h, 19 h und 60 h vorinkubiertes Aβ(1-42), welches auf 40 % (w/v) Iodixanol eingestellt wurde, um die Bedingungen des Dichtegradienten zu simulieren. Iodixanol eluiert deutlich vor Aβ(1-42) mit einer Retentionszeit zwischen 2 und 5 min. Das Chromatogramm dokumentiert deutlich die perfekte Abtrennung des die Detektion des Aβ im UV behindernden Iodixanols und die reproduzierbare Quantifizierung des Aβ-Peptids unabhängig davon, ob es in einer Probe als Monomer, Oligomer oder als Fibrille vorlag.

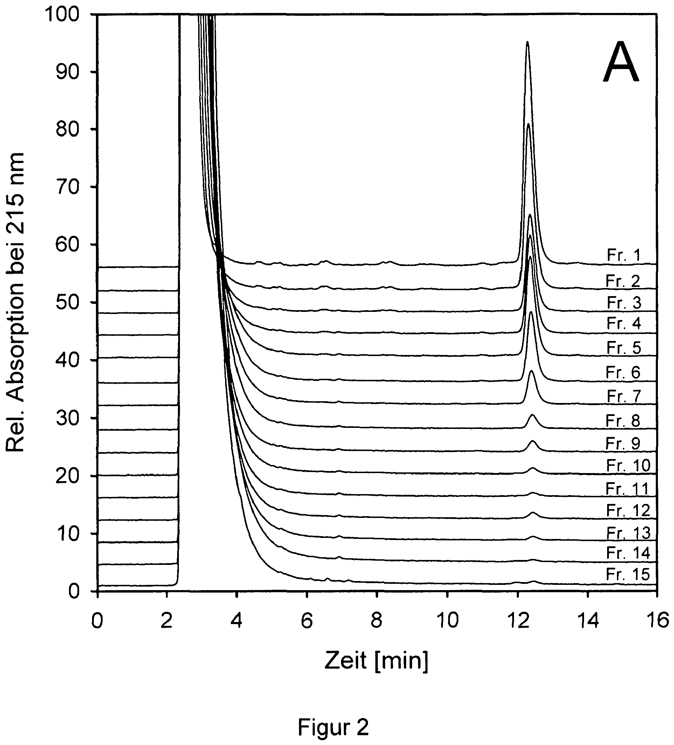

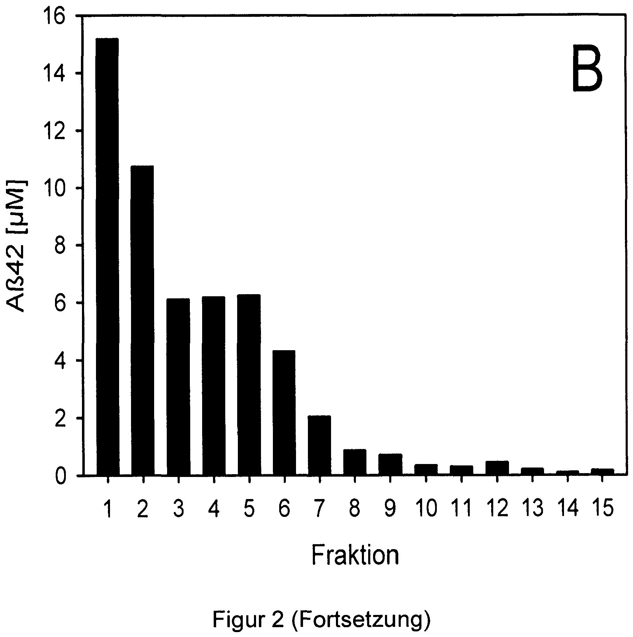

Figur 2 zeigt Chromatogramme der RP-HPLC für die Fraktionen 1 bis 15 eines Dichtegradienten mit den daraus berechneten Konzentrationen je Fraktion. Nach der Präparation einer mit Oligomeren angereicherten Aβ(1-42)-Probe wird diese auf einen Iodixanol-Dichtegradienten aufgeschichtet und mit 259.000 g bei 4 °C 3 h lang zentrifugiert. Dabei werden unterschiedliche Aβ-Aggregate nach ihrem s-Wert voneinander getrennt. Je größer der Partikel ist, desto weiter wandert er in den Gradienten ein. In den 14 von oben nach unten geernteten 140 μl großen Fraktionen und dem mit 60 μl 6 M GdnCl aufgekochten Pellet (Fraktion 15) wird die Konzentration des Aβ(1-42)-Peptids mittels RP-HPLC bestimmt. Hierzu werden 20 μl jeder Fraktion auf einer C8-Säule mit 30 % (v/v) Acetonitril und 0,1 % (v/v) Trifluoressigsäure bei einer Säulentemperatur von 80 °C vollständig denaturiert und nach Hydrophobizität aufgetrennt. Dabei wird das Aβ(1-42) vom Dichtegradientenmaterial Iodixanol, welches aufgrund seiner spektroskopischen Eigenschaften die Absorptionsmessung von Proteinen unmöglich macht, getrennt. Eluierendes Aβ(1-42) wird mittels UV-Absorption bei 215 nm detektiert. Peakflächenintegration erfolgt mittels Agilent Chemstation Software. Die Verrechnung daraus entstandener Werte mit der zuvor durchgeführten Kalibrierung erlaubt die Berechnung der Konzentration des in der jeweiligen Fraktion vorhandenen Aβ-Peptids. Wie zuvor in Figur 1 gezeigt sind hier nun die Elutionsverläufe für alle geernteten Fraktionen eines Dichtegradienten in einer Figur zusammengefasst (A). Wesentlich ist die Konstanz der Retentionszeit für das Aβ(1-42) unabhängig von der Position im Gradienten und damit von dem Aggregationszustand des Peptids. Im Diagramm (B) sind die anhand der für die Säule bestimmten Kalibrationsgeraden aus den Peakflächen errechneten Aβ-Konzentrationen in μmol/Liter dargestellt.

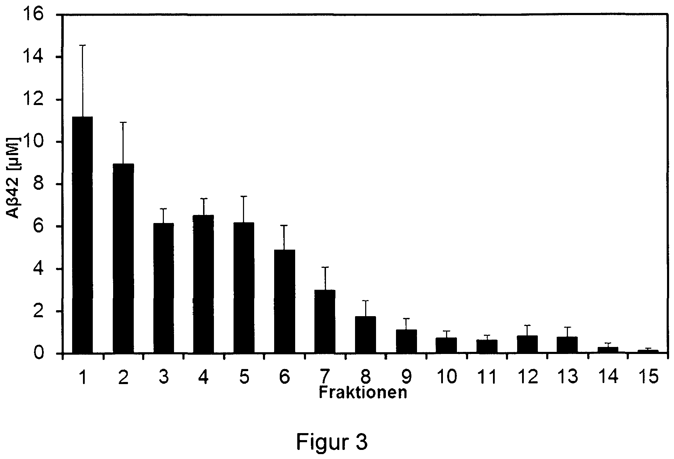

Figur 3 zeigt die gemittelte Größenverteilung für sechs Aβ(1-42)-Kontrollen ohne Wirkstoffzusatz nach Dichtegradientenzentrifugation und RP-HPLC. Pro Fraktion ist jeweils der Mittelwert aus sechs voneinander unabhängig durchgeführten Experimenten mit der sich ergebenen Standardabweichung dargestellt. In den ersten Fraktionen findet sich das Aβ(1-42)-Peptid wieder, welches zum Zeitpunkt der Fraktionierung als Monomer vorlag. Deutlich sind in den Fraktionen 4 bis 6 Oligomere des Aβ(1-42)-Peptids zu erkennen, welche während der Vorinkubation entstanden sind. Aufgrund ihrer Position im Gradienten und dem Vergleich mit Proteinen bekannter s-Werte haben diese einen s-Wert von ca. 5 S. Aufgrund des s-Wertes und unter der Annahme einer globulären Form sind diese Oligomere aus ca. 20 monomeren Einheiten aufgebaut. Kraftfeldmikroskopie-Analysen konnten die globuläre Form dieser Partikel bestätigen. Toxizitätstests mit den fraktionierten Oligomeren zeigten deren neurotoxische Eigenschaften. Durch die Kombination von Dichtegradienten-Fraktionierung und Konzentrationsbestimmung mit RP-HPLC wurde ein Verfahren entwickelt, welches erlaubt, den Einfluss potentieller Wirkstoffe auf den Anteil an einer toxischen Oligomerspezies des Aβ(1-42) Peptids Anteile von Aβ-Oligomeren in einer Probe sowie Einflüsse auf deren Quantität durch potentielle Wirkstoffe zu analysieren.

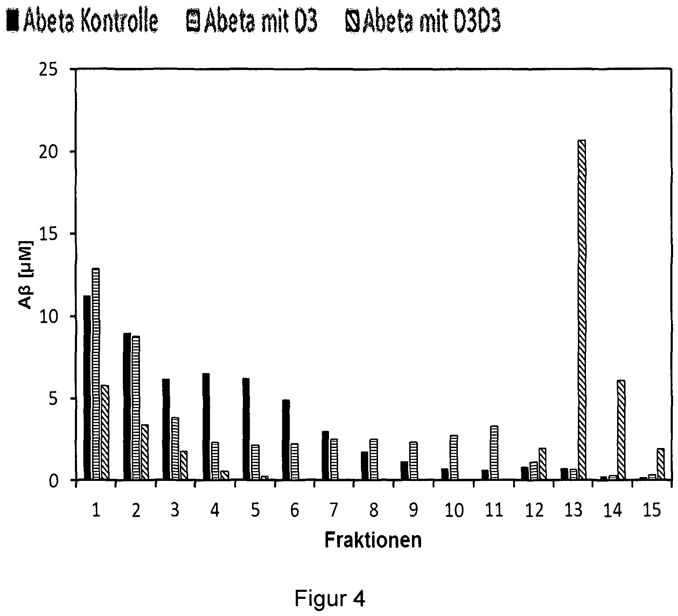

Figur 4 zeigt die Anwendung des vorgestellten Verfahrens auf die Analyse potentieller Wirkstoffe für die Therapie der Alzheimerschen Demenz am Beispiel eines D-Peptids (D3) und seines Tandem-Dimers (D3D3). Das Diagramm zeigt die Wirkung von 20 μM D3 gemäß SEQ ID NO: 1 und 10 μM D3D3, gemäß der SEQ ID NO: 2 auf die Größenverteilung der Aβ(1-42)-Aggregate in einem Dichtegradienten. Nach Vorbereitung einer oligomerreichen Aβ-Probe wird der zu testende Wirkstoff zugegeben. Dieser Ansatz wird nach einer 40 minütigen Inkubation auf einen Iodixanol-Dichtegradienten aufgeschichtet und mit 259.000 g bei 4 °C 3 h lang zentrifugiert. Als Kontrolle wird eine identisch behandelte Aβ(1-42)-Lösung ohne Wirkstoffzugabe eingesetzt. Durch die Zentrifugation werden unterschiedliche Aß-Aggregate nach ihrem Sedimentationskoeffizienten (s-Wert) aufgetrennt. Je größer der s-Wert der Partikel ist, desto weiter wandert er in den Gradienten ein. Anschließend wird der Gradient manuell von oben nach unten fraktioniert. Es resultieren 14 Fraktionen mit je140 μl und eine 60 μl Pelletfraktion, welche mit 60 μl 6 M GdnHCl aufgekocht wird. Aβ-Oligomere, bestehend aus ca. 20 monomeren Einheiten (s-Wert beträgt ca. 5 S), finden sich in den Fraktionen 4 bis 6 wieder. Für jeden Gradienten wird von allen Fraktionen jeweils ein 20 μl Aliquot mittels RP-HPLC analysiert. Zur Bestimmung der Aβ-Konzentration pro Fraktion wird Aß in dem Aliquot auf einer C8-Säule mit einer mobilen Phase bestehend aus 30 % (v/v) Acetonitril und 0,1 % (v/v) Trifluoressigsäure bei einer Säulentemperatur von 80 °C vollständig denaturiert und von dem Dichtegradientenmaterial Iodixanol und dem Wirkstoff abgetrennt, so dass es durch seine UV-Absorption bei 215 nm detektiert und quantifiziert werden kann. Die Datenaufzeichnung und Peakflächenintegration erfolgt unter der Verwendung der Agilent Chemstation Software (Agilent, Waldbronn). Die so gewonnenen Konzentrationsdaten sind in dem Diagramm gegen die Nummern der Fraktion aufgetragen. In den Fraktionen 4 bis 6, in denen sich oligomere Aβ-Spezies befinden, hat die Zugabe von D3 und D3D3 zu einer Verringerung der Menge an Oligomeren geführt. D3D3, welches nur halb so konzentriert eingesetzt worden war im Vergleich zu D3, zeigt in diesem Testsystem einen deutlich stärkeren Effekt als D3 in Bezug auf die Eliminierung oligomerer Aβ-Spezies. Der Anteil an großen Aggregaten in den Fraktionen 11 bis 14 ist unter dem Einfluss der Wirkstoffe, insbesondere bei D3D3 deutlich größer. Die Kombination vom Dichtegradienten und der RP-HPLC stellt somit ein neues Verfahren zum Screening von Wirkstoffen für eine Therapie der Alzheimerschen Demenz und zur quantitativen Charakterisierung amyloider und/oder aggregierender Peptide bzw. Proteine dar, welches als neues Selektionskriterium die Fähigkeit zur Eliminierung der Aβ-Oligomere verwendet.

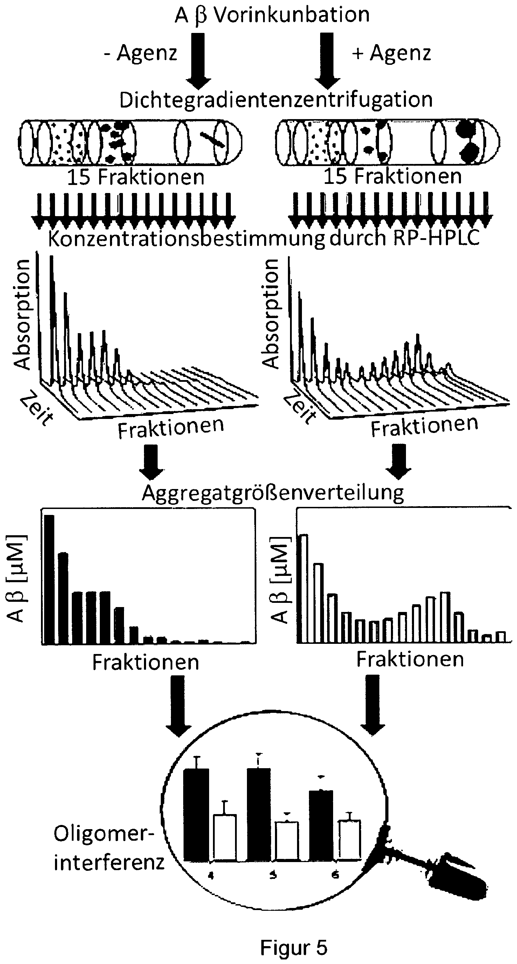

Figur 5 zeigt schematisch eine Ausführungsform des erfindungsgemäßen Verfahrens in Kürze. Nach Vorinkubation von Aβ, wird die Probe in einem Ansatz mit und ohne Wirkstoffzugabe weiter untersucht. Nach der Auftrennung in 15 Fraktionen mit Dichtegradientenzentrifugation wird nach der Denaturierung während der RP-HPLC die Konzentration an Monomerbausteinen in vorzugsweise allen 15 Fraktionen bestimmt und insbesondere der Einfluss des Wirkstoffs auf die Oligomerkonzentration ermittelt.

**Patentansprüche**

1. Verfahren zur quantitativen Charakterisierung amyloider und/oder aggregierender Peptide und/oder Proteine in einer Probe, mit den Schritten:

   - ein zu untersuchender Wirkstoff wird zu einer Probelösung gegeben, wobei die Probe bereitgestellt wurde und die Probe ein amyloides und/oder aggregierendes Peptid und/oder Protein mit mindestens einer Aggregatgröße und -Form umfasst,
   - die amyloiden und/oder aggregierenden Peptide und/oder Proteine werden in Hinblick auf ihre Aggregatgröße und -Form durch Dichtegradientenzentrifugation als Fraktionierung voneinander getrennt,
   - die Konzentrationsänderungen der Peptid- und/oder Proteinbausteine in mindestens einer Fraktion werden durch einen Vergleich mit Kontrollwerten ohne den Wirkstoff bestimmt.

2. Verfahren nach Anspruch 1,
   **dadurch gekennzeichnet, dass**
   die amyloiden und/oder aggregierten Peptide und/oder Proteine einer bestimmten Fraktion zu Monomerbausteinen denaturiert werden.

3. Verfahren nach vorherigem Anspruch,
   **gekennzeichnet durch**
   eine Kalibration der Fraktionierung, insbesondere durch eine Kalibration einer Dichtegradientenzentrifugation.

4. Verfahren nach einem der vorhergehenden Ansprüche,
   **dadurch gekennzeichnet, dass**
   die Konzentration der amyloiden und/oder aggregierten Peptide und/oder Proteine durch Umkehrphasen (RP-) HPLC bestimmt wird.

5. Verfahren nach einem der vorhergehenden Ansprüche,
   **dadurch gekennzeichnet, dass**
   durch die Wahl einer mobilen Phase und/oder durch die Wahl der Säulentemperatur der Umkehrphasen (RP-) HPLC gleichzeitig die Denaturierung der amyloiden und/oder aggregierten Peptide und/oder Proteine zu Monomeren bewirkt wird.

6. Verfahren nach einem der vorhergehenden Ansprüche,
   **dadurch gekennzeichnet, dass**
   eine mobile Phase für die Umkehrphasen HPLC gewählt wird, die auch die Trennung der amyloiden und/oder aggregierten Peptide und/oder Proteine von der den Dichtegradienten ausbildenden Substanz ermöglicht.

7. Verfahren nach einem der vorhergehenden Ansprüche,
   **dadurch gekennzeichnet, dass**
   während des Verfahrens eine Probe bereit gestellt wurde, in der aggregierendes $A\beta$, z. B. $A\beta(1-40)$, $A\beta(1-42)$, $A\beta(1-43)$, $A\beta(3-40)$, pyroGlu$A\beta(3-40)$ und pyroGlu$A\beta(3-42)$, oder Tau-Protein oder aber ein anderes typischerweise bei der Alzheimerschen Demenz vorkommendes Peptid mit tendenziell hohem Aggregationsverhalten gewählt wird.

8. Verfahren nach Anspruch 1 bis 7
   **gekennzeichnet dadurch, dass**
   der Einfluss des Wirkstoffes auch auf die Formverteilung der amyloiden und/oder aggregierten Peptide und/oder Proteine in mindestens einer Fraktion untersucht wird.

9. Verfahren nach vorherigem Anspruch,

**gekennzeichnet durch**

eine kraftfeldmikroskopische oder elektronenmikroskopische Untersuchung der Fraktionen.

**10.** Verfahren nach einem der vorhergehenden Ansprüche,
bei dem
die Konzentrationsänderung von Peptid- und/oder Proteinbausteinen in mehreren Fraktionen der Probe miteinander verglichen werden.

**11.** Verfahren nach dem vorherigem Anspruch,
**gekennzeichnet durch**,
einen Vergleich, der die Änderung der Konzentration an Monomeren und/oder Oligomeren und/oder Fibrillären und/oder anderen Konformeren einer Fraktion unter Einfluss des Wirkstoffs ermittelt und zu anderen Fraktionen in Beziehung setzt.

**12.** Verfahren nach einem der vorhergehenden Ansprüche,
**dadurch gekennzeichnet, dass**
ein geeigneter Wirkstoffkandidat für therapeutische Zwecke einer Krankheit, basierend auf einer Proteinfehlfaltungserkrankung, wie z. B. der Alzheimerschen Demenz, bereit gestellt wird, der nach seiner Zugabe wenige toxische Moleküle entstehen lässt.

**13.** Verfahren nach einem der vorhergehenden Ansprüche,
**dadurch gekennzeichnet, dass**
die amyloiden und/oder aggregierten Peptide und/oder Proteine in Hinblick auf Aggregatgröße und -Form zu mehreren, mindestens 2, vorzugsweise mindestens 3, besonders bevorzugt mindestens 4, 5, 6, 7, 8, 9, 10, 11, 12, 13, 14 oder 15 untersuchbaren Fraktionen voneinander getrennt werden.

**14.** Verfahren nach einem der vorhergehenden Ansprüche,
**dadurch gekennzeichnet, dass**
nach Zugabe eines Wirkstoffs die Konzentrationsänderung der Peptid- und/oder Proteinbausteine in mindestens einer Fraktion, vorzugsweise in mindestens 2, 3, 4, 5, 6, 7, 8, 9, 10, 11, 12, 13, 14 oder aber in allen untersuchbaren Fraktionen durch einen Vergleich mit Kontrollwerten ohne den Wirkstoff bestimmt wird.

**15.** Verfahren nach einem der vorhergehenden Ansprüche
**dadurch gekennzeichnet, dass**
solche Fraktionen umfasst werden, die vor der Auftrennung auch aggregierende bzw. aggregierte Peptide und/oder Proteine enthielten.

**Claims**

**1.** Method for the quantitative characterisation of amyloid and/or aggregating peptides and/or proteins in a sample with the steps:

- an active substance to be examined is put in a sample solution, in which the sample was provided and the sample comprises an amyloid and/or aggregating peptide and/or protein with at least one aggregate size and form, .
- the amyloid and/or aggregating peptides and/or proteins are separated from each other with regard to their aggregate size and form through density gradient centrifugation as fractionation,
- the changes in concentration of the peptide and/or protein components in at least one fraction are determined through a comparison with control values without the active substance.

**2.** Method according to claim 1,
**characterised in that**
the amyloid and/or aggregated peptides and/or proteins of a certain fraction are denatured into monomer components.

**3.** Method according to the previous claim,
**characterised by**

calibration of the fractionation, particularly through calibration of density gradient centrifugation.

4. Method according to one of the previous claims,
**characterised in that**
the concentration of the amyloid and/or aggregated peptides and/or proteins is determined through reversed-phase (RP) HPLC.

5. Method according to one of the previous claims,
**characterised in that**
denaturing the amyloid and/or aggregated peptides and/or proteins into monomers is caused by selecting a mobile phase and/or by selecting the column temperature of the reversed-phase (RP) HPLC.

6. Method according to one of the previous claims,
**characterised in that**
a mobile phase is selected for the reversed-phase HPLC, which also makes it possible to separate the amyloid and/or aggregated peptides and/or proteins from the substance forming the density gradients.

7. Method according to one of the previous claims,
**characterised in that**
during the method a sample was provided, in which aggregating $A\beta$, e.g. $A\beta$(1-40), $A\beta$(1-42), $A\beta$(1-43), $A\beta$(3-40), pyroGluA$\beta$(3-40) and pyroGluA$\beta$(3-42), or Tau protein or another peptide occuring typically in the case of Alzheimer's type dementia with a tendency to high aggregation behaviour is selected.

8. Method according to claim 1 to 7,
**characterised in that**
the influence of the active substance also on the form distrubution of the amyloid and/or aggregated peptides and/or proteins is examined in at least one fraction.

9. Method according to the previous claim,
**characterised by**
an atomic force microscope or electron microscope examination of the fractions.

10. Method according to one of the previous claims,
in which
the changes in concentration of peptide and/or protein components are compared with each other in several fractions of the sample.

11. Method according to the previous claim,
**characterised by**
a comparison, which determines the change in concentration in monomers and/or oligomers and/or fibrillar and/or other conformers of a fraction under the influence of the active substance and relates it to other fractions.

12. Method according to one of the previous claims,
**characterised in that**
a suitable active substance candidate for therapeutic purposes for a disease based on a protein misfolding disease, such as Alzheimer's type dementia for example, is provided, which allows fewer toxic molecules to occur after it is admininstered.

13. Method according to one of the previous claims,
**characterised in that**
the amyloid and/or aggregated peptides and/or proteins are separated from each other with regard to aggregate size and form into several, at least 2, preferably at least 3, particularly preferably at least 4, 5, 6, 7, 8, 9, 10, 11, 12, 13, 14 or 15 fractions that may be examined.

14. Method according to one of the previous claims,
**characterised in that**
after an active substance is administered the change in concentration of the peptide and/or protein components is determined through a comparison with control values without the active substance in at least one fraction, preferably in at least 2, 3, 4, 5, 6, 7, 8, 9, 10, 11, 12,13, 14 or in all fractions that may be examined.

15. Method according to one of the previous claims
    **characterised in that**
    such fractions are comprised, which before separation also contained aggregating or aggregated peptides and/or proteins.

**Revendications**

1. Procédé de caractérisation quantitative de peptides et/ou protéines amyloïdes et/ou agrégés dans un échantillon, comportant les étapes suivantes :

   - un principe actif à analyser est ajouté à une solution d'échantillon, dans lequel l'échantillon a été mis à disposition et l'échantillon comprend un peptide et/ou une protéine amyloïdes et/ou agrégés avec au moins une dimension et une forme d'agrégat,
   - les peptides et/ou protéines amyloïdes et/ou agrégés sont séparés les uns des autres eu égard à leur dimension et leur forme d'agrégat, par centrifugation en gradient de densité en tant que fractionnement,
   - les modifications de concentration des composants peptidiques et/ou protéiques dans au moins une fraction sont déterminées par une comparaison avec des valeurs témoins sans le principe actif.

2. Procédé selon la revendication 1,
   **caractérisé en ce que**
   les peptides et/ou protéines amyloïdes et/ou agrégés d'une fraction déterminée sont dénaturés en composants de monomère.

3. Procédé selon une revendication précédente,
   **caractérisé par**
   un étalonnage du fractionnement, en particulier par un étalonnage d'une centrifugation en gradient de densité.

4. Procédé selon l'une des revendications précédentes,
   **caractérisé en ce que**
   la concentration des peptides et/ou protéines amyloïdes et/ou agrégés est déterminée par HPLC en phases inverses (RP).

5. Procédé selon l'une des revendications précédentes,
   **caractérisé en ce que**
   par le choix d'une phase mobile et/ou par le choix de la température de colonne de la HPLC en phases inverses (RP) est obtenue en même temps la dénaturation des peptides et/ou protéines amyloïdes et/ou agrégés en monomères.

6. Procédé selon l'une des revendications précédentes,
   **caractérisé en ce que**
   une phase mobile est choisie pour la HPLC en phases inverses, qui rend possible également la séparation des peptides et/ou protéines amyloïdes et/ou agrégés de la substance constituant les gradients de densité.

7. Procédé selon l'une des revendications précédentes,
   **caractérisé en ce que**
   pendant le procédé, un échantillon a été mis à disposition, dans lequel une protéine Aβ agrégée, par exemple, Aβ(1-40), Aβ(1-42), Aβ(1-43), Aβ(3-40), pyroGluAβ(3-40) et pyroGluAβ(3-42), ou Tau ou un autre peptide typiquement présent dans la maladie d'Alzheimer avec une tendance élevée de comportement d'agrégation est choisie.

8. Procédé selon une revendication 1 à 7,
   **caractérisé en ce que**
   l'effet du principe actif également sur la distribution des formes des peptides et/ou protéines amyloïdes et/ou agrégés est analysé dans au moins une fraction.

9. Procédé selon une revendication précédente,

**caractérisé par**

une analyse par microscopie à champ de force ou microscopie électronique des fractions.

10. Procédé selon l'une des revendications précédentes,
dans lequel
les modifications de la concentration des composants peptidiques et/ou protéiques sont comparées les unes aux autres dans plusieurs fractions de l'échantillon.

11. Procédé selon la revendication précédente,
**caractérisé par**
une comparaison qui établit la modification de la concentration en monomères et/ou oligomères et/ou fibrillaires et/ou autres conformères d'une fraction sous l'effet du principe actif et met en rapport d'autres fractions.

12. Procédé selon l'une des revendications précédentes,
**caractérisé en ce que**
un principe actif candidat approprié est mis à disposition à des fins thérapeutiques d'une maladie, sur la base d'une maladie liée à un défaut de repliement d'une protéine, par exemple la maladie d'Alzheimer, qui permet de produire des molécules moins toxiques après son administration.

13. Procédé selon l'une des revendications précédentes,
**caractérisé en ce que**
les peptides et/ou protéines amyloïdes et/ou agrégés sont séparés les uns des autres eu égard à leur dimension et leur forme d'agrégat en plusieurs, au moins 2, de préférence au moins 3, de manière particulièrement préférée au moins 4, 5, 6, 7, 8, 9, 10, 11, 12, 13, 14 ou 15 fractions analysables.

14. Procédé selon l'une des revendications précédentes,
**caractérisé en ce que**
après l'administration d'un principe actif, la modification de concentration des composants peptidiques et/ou protéiques est déterminée dans au moins une fraction, de préférence dans au moins 2, 3, 4, 5, 6, 7, 8, 9, 10, 11, 12, 13, 14 ou également dans toutes les fractions analysables par une comparaison avec des valeurs témoins sans le principe actif.

15. Procédé selon l'une des revendications précédentes,
**caractérisé en ce que**
sont comprises les fractions qui contenaient avant la séparation également des peptides et/ou des protéines s'agrégeant ou agrégés.

Figur 1

Figur 2

Figur 2 (Fortsetzung)

Figur 3

■Abeta Kontrolle  ⊟Abeta mit D3  ▨Abeta mit D3D3

Figur 4

A β Vorinkunbation

- Agenz                    + Agenz

Dichtegradientenzentrifugation

15 Fraktionen          15 Fraktionen

Konzentrationsbestimmung durch RP-HLPC

Absorption          Absorption

Zeit                Zeit

Fraktionen          Fraktionen

Aggregatgrößenverteilung

A β [µM]            A β [µM]

Fraktionen          Fraktionen

Oligomer-
interferenz

Figur 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **DEGUO DU ; AMBER N. MURRAY ; EHUD COHEN ; HYUN-EUI KIM ; RYAN SIMKOVSKY ; ANDREW DILLIN ; JEFFERY W. KELLY.** A Kinetic Aggregation Assay Allowing Selective and Sensitive Amyloid-β Quantification in Cells and Tissues. *Biochemistry,* 2011, vol. 50, 1607-1617

- **S. A. FUNKE ; T. VAN GROEN ; I. KADISH ; D. BARTNIK ; L. NAGEL-STEGER ; O. BRENER ; T. SEHL ; R. BATRA-SAFFERLING ; C. MORISCOT ; G. SCHOEHN.** Oral Treatment with the D-Enantiomeric Peptide D3 Improves the Pathology and Behavior of Alzheimer's Disease Transgenic Mice. *ACS Chem. Neurosci.,* 2010, vol. 1, 639-648

SEQUENCE LISTING

<110> Forschungszentrum Juelich GmbH

<120> verfahren zur quantitativen Charakterisierung amyloider und / oder aggregierender Peptide und / oder Proteine in einer Probe

<130> PT 1.2626 PCT

<160> 2

<170> PatentIn version 3.5

<210> 1
<211> 12
<212> PRT
<213> Artificial sequence

<220>
<223> Artificial Sequence

<400> 1

```
Arg Pro Arg Thr Arg Leu His Thr His Arg Asn Arg
1               5               10
```

<210> 2
<211> 24
<212> PRT
<213> Artificial sequence

<220>
<223> Artificial Sequence

<400> 2

```
  Arg Pro Arg Thr Arg Leu His Thr His Arg Asn Arg Arg Pro Arg Thr
   1                   5                   10                  15

  Arg Leu His Thr His Arg Asn Arg
                   20
```